# EUROPEAN PATENT APPLICATION

(11) **EP 3 524 994 A1**
(43) Date of publication of application: **14.08.2019**
(21) Application number: 18155685.3
(22) Date of filing: 08.02.2018
(51) Int. Cl.: G01R 33/385, G01R 33/54

(54) **MRI WITH ACOUSTIC SOUND IN PRE-DATA-ACQUISITION-MODE**

(71) Applicant: Koninklijke Philips N.V., 5656 AE Eindhoven (NL)
(72) Inventor: FORTHMANN, Peter, 5656 AE Eindhoven (NL)
(74) Representative: de Haan, Poul Erik

(57) **Abstract**

The invention relates to a magnetic resonance imaging system (100) comprising a main magnet (104), a magnetic field gradient system with antenna elements (114), a radio-frequency system, a memory (136) storing machine executable instructions comprising a first set of machine executable instructions (150) for operating the magnetic resonance imaging system (100) in a pre-data-acquisition-mode in which data acquisition by the antenna elements (114) is switched off, and a processor (130) for controlling the magnetic resonance imaging system (100). An execution of the first set of machine executable instructions (150) causes the processor (130) to control the magnetic resonance imaging system (100) to generate in the pre-data-acquisition-mode acoustic sound using the magnetic field gradient system. The generation of the acoustic sound comprises generating an alternating gradient magnetic field by the magnetic field gradient system.

## Description

### FIELD OF THE INVENTION

The invention relates to magnetic resonance imaging system, in particular it relates to magnetic resonance imaging system, a computer program product and a method for generating acoustic sound with a magnetic field gradient system in a pre-data-acquisition-mode.

### BACKGROUND OF THE INVENTION

Due to strong alternating magnetic fields which are generated during data acquisition by a magnetic resonance imaging (MRI) system, hammering acoustic sounds are generated by Lorentz forces acting on current-carrying components of the MRI system.

Most patients are unused to this type and volume of noise. Therefore, patients may perceive the acoustic sound generated by the MRI system during data acquisition as uncomfortable and even scary. In a large group of patients, acoustic sound generated during MRI exams is known to elicit feelings ranging from uneasiness to fear. In addition, especially for children, the dimensions of a MRI system alone bear potential for apprehension. Additional unfamiliar hammering gradient sounds during the data acquisition may be perceived as nothing short of frightening.

Patients who feel uncomfortable may be tense, restless and not relaxed. Such a restlessness may for example result in movements which lead to a low image quality due to motion artifacts. In particular, an unexpected hammering sound may make the patient flinch.

Thus, there is a need to reduce negative implications of acoustic sounds generated by the MRI system during data acquisition on the patient and consequently on the image quality.

### SUMMARY OF THE INVENTION

The invention provides for a magnetic resonance imaging system, a method of operating the magnetic resonance imaging system, and a computer program product in the independent claims. Embodiments are given in the dependent claims.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a 'circuit', 'module', or 'system'. Furthermore, aspects of the present invention may take the form of a computer program product embodied in one or more computer readable medium(s) having computer executable code embodied thereon.

Any combination of one or more computer readable medium(s) maybe utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising 'a processor' should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the 'C' programming language or similar programming languages and compiled into machine executable instructions. In some instances, the computer executable code may be in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection maybe made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance Imaging (MRI) data, also referred to as Magnetic Resonance (MR) data, is defined herein as being the recorded measurements of radio frequency signals emitted by nuclear spins using antenna elements of a magnetic resonance imaging system during a magnetic resonance data acquisition process, also referred to as a magnetic resonance imaging scan. A Magnetic Resonance Imaging (MRI) image or MR image is defined herein as being the reconstructed two or three-dimensional visualization of anatomic data comprised by the magnetic resonance imaging data. This visualization may be performed using a computer. Magnetic resonance imaging data maybe provided using a representation of the respective data in k-space or image space. Using a Fourier transformation, the magnetic resonance imaging data may be transformed from k-space to image space or vice versa.

In one aspect, the invention relates to magnetic resonance imaging system. The magnetic resonance imaging system comprises a main magnet configured for generating a spatially and temporally constant main magnetic field within an imaging zone, a magnetic field gradient system comprising a set of gradient coils configured for generating a spatially and temporally alternating gradient magnetic field within the imaging zone, a radio-frequency system comprising a set of antenna elements configured for acquiring magnetic resonance data from the imaging zone, a memory storing machine executable instructions comprising a first set of machine executable instructions for operating the magnetic resonance imaging system in a pre-data-acquisition-mode in which data acquisition by the antenna elements is switched off, and a processor for controlling the magnetic resonance imaging system, wherein an execution of the first set of machine executable instructions causes the processor to control the magnetic resonance imaging system to generate in the pre-data-acquisition-mode acoustic sound using the magnetic field gradient system. The generation of the acoustic sound comprises generating the alternating gradient magnetic field by the magnetic field gradient system.

Embodiment may have the beneficial effect of introducing the patient before the data acquisition to the acoustic sound to be expected during data acquisition, rather than exposing him or her to the gradient coil acoustic sound all at once, when he or she is expected to lay still in order to avoid motion artifacts.

The acoustic sound emitted by the gradient coils may be a significant source of patient discomfort and has the potential to scare people. In case a patient feels uncomfortable and is restless or even flinches due to unexpected acoustic sounds, there is a high likelihood of undesired movements which may cause motion artifacts. Image degradation of magnetic resonance images due to subject motion during the acquisition of magnetic resonance data is one of the most persistent problems in the clinical application of magnetic resonance imaging. The associated motion artifacts may e.g. appear as ghosting or blurring in the images and often reduce image quality to a degree that makes medical analysis impossible.

Due to lack of methods for automatic motion artifact detection, the evaluation of magnetic resonance images currently relies on manual labeling of artifact levels by an experienced radiologist. However, such an approach generates considerable additional workload and may strongly depend on the experience of individual person evaluating the images. Furthermore, in case image degradation is to strong, i.e. the artifact level is too high, the full data acquisition may have to be repeated.

Embodiments may have the beneficial effect of reducing patient stress in general and reducing stress-induced patient motion in particular. Thus, the likelihood of undesired movements of a patient during data acquisition due to acoustic sounds generated by the MRI system may be minimized. Patients, especially children, to be scanned are enabled to get acquainted with the acoustic sounds of the MRI system to be expected during data acquisition. Thus, the patients may be prepared for the potential pending stress caused by gradient coil acoustic sounds by being provided with a preview of what is going to happen. This way the patients may get primed and acquainted to what lies ahead of them. Such a preparation in form of an operation of the MRI system in a pre-data-acquisition-mode before data acquisition may take away much of the surprise, when the magnetic field gradient system is eventually firing at full blast, i.e. pulses.

According to embodiments, the MRI system is operated in the pre-data-acquisition-mode during the patient preparation time, e.g. from when he or she enters the room with the MRI system until the data acquisition starts. EEmbodiments may have the beneficial effect of sustainably reducing the patient's anxiety during the preparation phase. A preparation for what is to come takes the surprise away and can prevent an elevation of the patient's stress level during the exam. A lower stress level is a benefit for the patient and can also be a benefit for imaging, due to less potentially stress-induced motion artifacts.

According to embodiments, the generating of the acoustic sound further comprises generating simultaneously with the alternating gradient magnetic field the constant main magnetic field using the main magnet. Embodiment may have the beneficial effect of generating a realistic acoustic sound during the operation of the MRI system in the pre-data-acquisition-mode taking into account the influence of the main magnetic field on the acoustic sound generated during real data acquisition, in particular regarding the sound volume. The acoustic sound generated by the MRI system during data acquisition is mainly due to the gradient coils generating a temporally alternating gradient magnetic field. In order to generate the alternating gradient magnetic field, rapid alternations of currents are caused within the gradient coils. These alternating currents, in the presence of the strong main magnetic field of the MRI system, result in significant Lorentz forces that act upon the gradient coils. The resulting acoustic sound may e.g. be manifested as loud tapping, knocking, chirping, squeaking sounds, or other sounds being produced, when the Lorentz forces cause a motion or vibration of the gradient coils as they impact against their mountings within the MRI system which, in turn, flex and vibrate. These vibrations may be radiated into the air as sound waves or transmitted by physical linages to other parts of MRI system. In a three-tesla system, a strength common in clinical practice, acoustic sounds may e.g. be as loud as 125 decibels.

According to embodiments, the memory further comprises pulse sequence commands configured for controlling the magnetic resonance imaging system to acquire the magnetic resonance data from the imaging zone. The alternation of the gradient magnetic field in the pre-data-acquisition-mode is executed according to a gradient magnetic field alternating scheme defined by the pulse sequence commands. Embodiment may have the beneficial effect of generating a more realistic acoustic sound during the operation of the MRI system in the pre-data-acquisition-mode. Each pulse sequence used for acquiring magnetic resonance data may produce a characteristic sound pattern based on its gradient waveforms. Echo-planar sequences may be among the loudest, producing maximum sound pressures in the range of 110 to 120 dB. By alternating the gradient magnetic field in the pre-data-acquisition-mode according to a gradient magnetic field alternating scheme defined by pulse sequence commands which will later on be used for the real, i.e. actual, data acquisition, may better prepare the patient for what he has to expect during the scan.

According to embodiments, amplitudes of pulses generated by the magnetic field gradient system in the pre-data-acquisition-mode are reduced relatively to amplitudes defined for the pulses by the gradient magnetic field scheme. According to embodiments, the amplitudes of pulses generated by the magnetic field gradient system are gradually raised until an amplitude threshold is reached. Embodiment may have the beneficial effect of allowing for a slow and gradual introduction of the patient to the type of acoustic sound he or she has to expect without risking to surprise the patient by a sudden high volume.

According to embodiments, the amplitude threshold is defined by a pulse with maximum amplitude defined by the gradient magnetic field scheme for the magnetic field gradient system. Embodiment may have the beneficial effect that at the end of the pre-data-acquisition-mode, when the amplitude threshold is reached, the acoustic sound may be highly realistic not only in frequency and rhythm, but also regarding volume.

According to embodiments, the acoustic sound generated using the gradient coils further comprises a melodic tune. Embodiment may have the beneficial effect of providing a combination of sounds during the pre-data-acquisition-mode, which due to the melodic tune is experienced by the patient as being more pleasant than the pure realist sound of the MRI system, i.e. the sound generated during data acquisition. Thus, the patient maybe enabled to better relax before the data acquisition. Making the machine hum a melodic tune may further help to mitigate its menacing appearanc. The melodic tune may e.g. comprise a regular recurring rhythmic pattern, in particular a slow rhythmic pattern. The melodic tune may be a tune pleasant for the patient. According to embodiments, the patient may for example be enable to select the melodic pattern from a set of melodic example patterns in advance or at the beginning of the operation in the pre-data-acquisition-mode. Thus, a patient individual melodic tune may be provided. According to embodiments, the melodic tune may, at least temporarily, be dominating over the pure realist sound of the MRI system.

According to embodiments, the volume of the acoustic sound is gradually raised until a volume threshold is reached. According to embodiments, the volume threshold is defined by the volume of the acoustic sound generated by the pulse with maximum amplitude defined by the gradient magnetic field scheme for the magnetic field gradient system. Embodiment may have the beneficial effect of gradually introducing the patient to the real acoustic sound to be expected during an MRI scan. For example, the sound generation may start shortly after the patient enters the room with the MRI system and the operator explains to the patient what is going to happen. The sound may get louder automatically until the volume threshold is reached.

According to embodiments, the magnetic resonance imaging system further comprises a user interface configured for manually tuning the acoustic sound generated by the magnetic field gradient system in the pre-data-acquisition-mode. Embodiment may have the beneficial effect of enabling the patient to experience the acoustic sound to be expected during data acquisition on his or her own. In particular, the patient, especially a child, may experience a feeling of control regarding the sound. Thus, the patient to be examined may get acquainted with the acoustic sounds of the MRI system in a playful way. For example, the patient may be offered the display, e.g. in form of a handheld device, like e.g. a tablet, in order to play with the sound, e.g. regarding the volume. The user interface may be connected with the MRI system via wire or wireless. The user interface may e.g. display a virtual piano keyboard or the like on a touchscreen for the patient to play on. According to embodiments, the patient may be enabled to select via the user interface a melodic tune from a set of melodic tunes which is to be mixed with the realistic acoustic sounds in the pre-data-acquisition-mode.

According to embodiments, the machine executable instructions stored in the memory comprise a second set of machine executable instructions for operating the magnetic resonance imaging system in a data-acquisition-mode in which data acquisition by the antenna elements is switched on. An execution of the second set of machine executable instructions causes the processor to control the magnetic resonance imaging system using the pulse sequence commands to acquire magnetic resonance data from the imaging zone using the antenna elements and to reconstruct a magnetic resonance image using the acquired magnetic resonance data.

Embodiment may have the beneficial effect of acquiring magnetic resonance data during operation in the data-acquisition-mode, after the patient has been prepared for what is to be expected regarding acoustic sound generated during in the data-acquisition-mode. Thus, higher quality magnetic resonance images may be obtained due to a reduction of motion artifacts compared to a data acquisition without pre-data-acquisition-mode.

In a further aspect, the invention relates to a computer program product comprising machine executable instructions for execution by a processor controlling a magnetic resonance imaging system. The magnetic resonance imaging system further comprises a main magnet configured for generating a spatially and temporally constant main magnetic field within an imaging zone, a magnetic field gradient system comprising a set of gradient coils configured for generating a spatially and temporally alternating gradient magnetic field within the imaging zone, and a radio-frequency system comprising a set of antenna elements configured for acquiring magnetic resonance data from the imaging zone. The machine executable instructions comprise a first set of machine executable instructions for operating the magnetic resonance imaging system in a pre-data-acquisition-mode in which data acquisition by the antenna elements is switched off. An execution of the first set of machine executable instructions causes the processor to control the magnetic resonance imaging system to generate in the pre-data-acquisition-mode acoustic sound using magnetic field gradient system. The generation of the acoustic sound comprises generating the alternating gradient magnetic field by the magnetic field gradient system.

According to embodiments the computer program product comprises machine executable instructions which are configured to enable the processor of the MRI system to control the same to implement any of the aforementioned functional features of the MRI system.

In a further aspect, the invention relates to a method of operating a magnetic resonance imaging system. The magnetic resonance imaging system comprises a main magnet configured for generating a spatially and temporally constant main magnetic field within an imaging zone, a magnetic field gradient system comprising a set of gradient coils configured for generating a spatially and temporally alternating gradient magnetic field within the imaging zone, a radio-frequency system comprising a set of antenna elements configured for acquiring magnetic resonance data from the imaging zone, a memory storing machine executable instructions comprising a first set of machine executable instructions for operating the magnetic resonance imaging system in a pre-data-acquisition-mode in which data acquisition by the antenna elements is switched off, and a processor for controlling the magnetic resonance imaging system. The method comprises executing the first set of machine executable instructions by the processor and generating in the pre-data-acquisition-mode acoustic sound using the magnetic field gradient system. The generation of the acoustic sound comprises generating the alternating gradient magnetic field by the magnetic field gradient system.

According to the method comprises controlling the MRI system to implement any of the aforementioned functional features of the MRI system.

### BRIEF DESCRIPTION OF THE DRAWINGS

In the following preferred embodiments of the invention will be described, by way of example only, and with reference to the drawings in which:
Fig. 1 illustrates an example of a magnetic resonance imaging system and
Fig. 2 illustrates an example of a method of operating a magnetic resonance imaging system.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

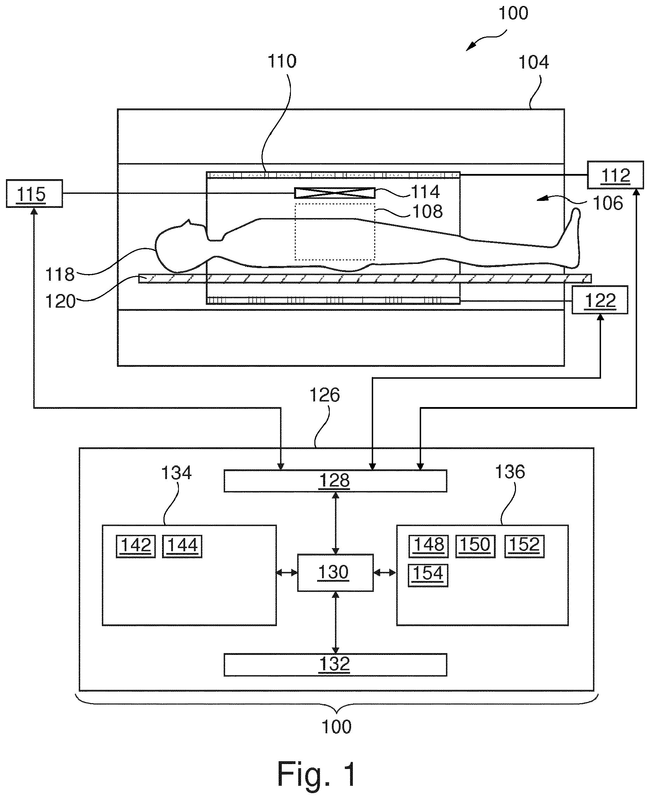

Fig. 1 shows an example of a magnetic resonance imaging system 100 with a magnet 104. The main magnet 104 is a superconducting cylindrical type magnet 104 with a bore 106 through it. The use of different types of magnets is also possible. For instance, it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 106 of the cylindrical magnet 104 there is an imaging zone 108 where the magnetic field is strong and uniform enough to perform magnetic resonance imaging.

Within the bore 106 of the magnet there is also a set of magnetic field gradient coils 110 forming a magnetic field gradient system which is used for acquisition of magnetic resonance data to spatially encode magnetic spins within the imaging zone 108 of the magnet 104. The magnetic field gradient coils 110 connected to a magnetic field gradient coil power supply 112. The magnetic field gradient coils 110 are intended to be representative. Typically, magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 is a radio-frequency coil 114, also referred to as radio-frequency antenna system, for manipulating the orientations of magnetic spins within the imaging zone 108 and for receiving radio transmissions from spins also within the imaging zone 108. The radio frequency coil 114 may contain multiple coil elements. The radio-frequency coil 114 is connected to a radio frequency transceiver 115. The radio-frequency coil 114 and radio frequency transceiver 115 may be replaced by separate transmit and receive coils and a separate transmitter and receiver. It is understood that the radio-frequency coil 114 and the radio frequency transceiver 115 are representative. The radio-frequency coil 114 is intended to also represent a dedicated transmit antenna and a dedicated receive antenna. Likewise, the transceiver 115 may also represent a separate transmitter and receivers. The radio-frequency coil 114 may also have multiple receive/transmit elements and the radio frequency transceiver 115 may have multiple receive/transmit channels. In case of separate transmit antenna and receive antenna, e.g. only the receive antenna may be switched off in the pre-data-acquisition-mode, while the transmit antenna may be switched on. In case antenna elements 114 are configured as combined receive and transmit antenna, e.g. only the receive function may be switched off in the pre-data-acquisition-mode, while the transmit function may be switched on. For example, signals received during the pre-data-acquisition-mode may not be processed by the magnetic resonance imaging system 100.

The subject support 120 is attached to an optional actuator 122 that is able to move the subject support and the subject 118 through the imaging zone 108. In this way, a larger portion of the subject 118 or the entire subject 118 can be imaged. The transceiver 115, the magnetic field gradient coil power supply 112 and the actuator 122 are shown as being connected to a hardware interface 128 of computer system 126.

The computer 126 is further shown as containing a processor 130 which is operable for executing machine-readable instructions. The computer 126 is further shown as comprising a user interface 132, computer storage 134 and computer memory 136 which are all accessible and connected to the processor 130.

The user interface 132 may be provided in form of a monitor. The user interface 132 may be provided as one of several user interfaces 132 at least one of which is provided in order to enable the patient to manually tune acoustic sounds generated by the magnetic resonance imaging system 100 in the pre-data-acquisition-mode. The user interface 132 for tuning the acoustic sound may e.g. be provided in form of a mobile handheld device, e.g. a tablet. The interfaces 132 may be operatively connected with the processor 130 via a wire or wireless.

The computer storage 134 may contain magnetic resonance data 142 acquired by the magnetic resonance imaging system 100 which is controlled by the processor 130 executing one or more of the pulse sequences 148. The acquired magnetic resonance data 142 may be used to reconstruct one or more magnetic resonance images 144.

The computer memory 136 may contain one or more pulse sequences 148. The pulse sequences 148 are either instructions or data which can be converted into instructions which enable the processor 130 to acquire magnetic resonance data 142 using the magnetic resonance imaging system 100.

The computer memory 136 may further comprising a first control module 150. The first control module 150 may contain computer executable code or instructions, i.e. a first set of machine executable instructions, which enable the processor 130 to control the operation of the magnetic resonance imaging system 100 in the pre-data-acquisition-mode in which the data acquisition by the antenna elements 114 is switched off, while acoustic sound is generated using the magnetic field gradient coils 110.

The computer memory 136 may also comprising a second control module 150. The second control module 152 may contain computer executable code or instructions, i.e. a second set of machine executable instructions, which enable the processor 130 to control the operation of the magnetic resonance imaging system 100 in the data-acquisition-mode in which the magnetic resonance imaging system 100 acquires the magnetic resonance data 142. For instance, the second control module 152 may work in conjunction with the pulse sequences 148 to acquire the magnetic resonance imaging data 142. The computer storage 136 is shown as further containing an imaging reconstruction module 154 which contains computer executable code or instructions which enable the processor 130 to control the operation and function of the magnetic resonance imaging system 100 to reconstruct magnetic resonance images 144 using the acquired magnetic resonance data 142.

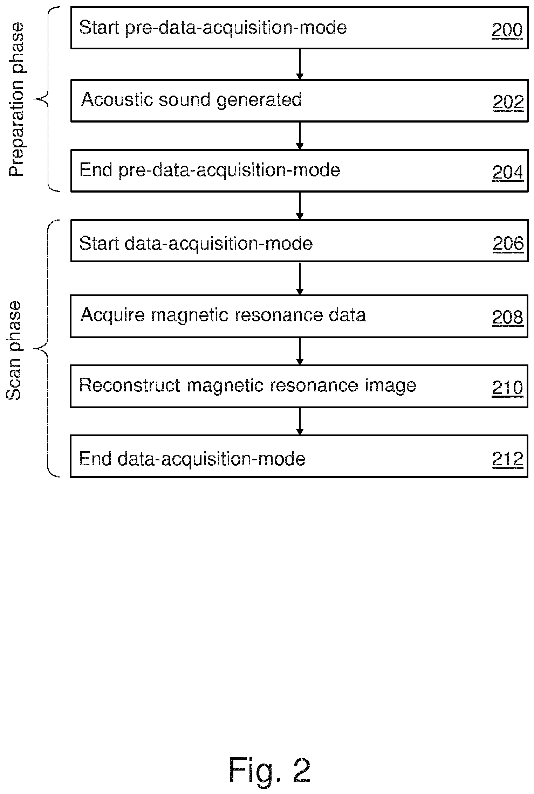

Fig. 2 shows a schematic flowchart which illustrates a method of operating the magnetic resonance imaging system. The method may be divided in two phases, a first phase, i.e. a preparation phase, in which the patient is prepared for the MRI scan and data acquisition, and a second phase, i.e. a scan phase, in which the MRI scan is executed and magnetic resonance data is acquired. In step 200, at the beginning of the preparation phase, the operation of the MRI system in the pre-data-acquisition-mode is started. The operation of the data-acquisition-mode may e.g. start, when the patient enters the room in which the MRI system is located and the operator explains to the patient what will happen. The volume of the acoustic sound generated using the magnetic field gradient system may start at a low level and be gradually raised until a volume threshold is reached. The volume threshold may e.g. be equal to the maximum volume of the acoustic sound generated by the MRI system during data acquisition and the MRI system is controlled according to a pulse sequence configured for an acquisition of magnetic resonance data from the imaging zone during the scan phase. According to embodiment, acoustic sound in form of a melodic tune may be generated using the magnetic field gradient system. The melodic tune may be mixed with the realistic acoustic sound resulting from an execution of a pulse sequence. According to embodiments, the acoustic sound of the melodic tune may start at a higher volume than the acoustic sound resulting from the execution of the pulse sequence. According to embodiments, the acoustic sound of the melodic tune may be generated at the beginning. Then, the acoustic sound of the melodic tune and/or the acoustic sound resulting from the execution of the pulse sequence may be raised. According to embodiments, the volume of the acoustic sound resulting from the execution of the pulse sequence may be raised faster than the acoustic sound of the melodic tune. According to embodiments, the volume of the acoustic sound of the melodic tune may be reduced or kept at a constant level, while the volume of the acoustic sound resulting from the execution of the pulse sequence is raised. Thus, the acoustic sound resulting from the execution of the pulse sequence may be raised above the level of the volume of the acoustic sound of the melodic tune. According to alternative embodiments, both the acoustic sound resulting from the execution of the pulse sequence as well as the acoustic sound of the melodic tune may end up with the same volume. In step 204, at the end of the preparation phase, the operation of the magnetic resonance imaging system in the pre-data-acquisition-mode ends.

In step 206, at the beginning of the scan phase, the operation of the magnetic resonance imaging system in the data-acquisition-mode is started. The data-acquisition-mode may e.g. start, when the patient enters the imaging zone and/or when the actual data acquisition starts with the execution of the pulse sequence configured for controlling the MRI system to acquire magnetic resonance data from the imaging zone. In step 208, magnetic resonance data is acquired from the imaging zone using the antenna elements. In step 210, the acquired magnetic resonance data is used to reconstruct one or more magnetic resonance images. During data acquisition, the MRI system necessarily generates acoustic sound due to the strong alternating magnetic fields required for acquired magnetic resonance data with sufficient precision. However, since the patient has been introduced to this type of acoustic sound during the preparation phase and had time to get accustomed to it, he or she may be more relaxed and less surprised or even scared of the sound. Therefore, the likelihood of undesired motion resulting in motion artifacts may be reduced. Consequently, the quality of the acquired data as well as the resulting magnetic resonance images may be improved. Furthermore, a repetition of data acquisition due to an insufficient level of quality maybe avoided. In step 212, at the end of the scan phase, the operation of the magnetic resonance imaging system in the data-acquisition-mode ends. According to embodiments, the order of steps 210 and 212 may be altered, i.e. according to embodiments the scan phase may end after acquisition of sufficient data and the reconstruction of magnetic resonance images may be executed later on, e.g. during a separate evaluation phase.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word 'comprising' does not exclude other elements or steps, and the indefinite article 'a' or 'an' does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: magnetic resonance imaging system
- 104: main magnet
- 106: bore of magnet
- 108: imaging zone
- 110: magnetic field gradient coil
- 112: magnetic field gradient coil power supply
- 114: radio-frequency coil
- 115: transceiver
- 118: subject
- 120: subject support
- 122: actuator
- 126: computer system
- 128: hardware interface
- 130: processor
- 132: user interface
- 134: computer storage
- 136: computer memory
- 142: magnetic resonance data
- 144: magnetic resonance image
- 148: pulse sequences
- 150: first control module
- 152: second control module
- 154: imaging reconstruction module

## Claims

1. A magnetic resonance imaging system (100) comprising:
a main magnet (104) configured for generating a spatially and temporally constant main magnetic field within an imaging zone (108),
a magnetic field gradient system comprising a set of gradient coils (110) configured for generating a spatially and temporally alternating gradient magnetic field within the imaging zone (108),
a radio-frequency system comprising a set of antenna elements (114) configured for acquiring magnetic resonance data (142) from the imaging zone (108),
a memory (136) storing machine executable instructions comprising a first set of machine executable instructions (150) for operating the magnetic resonance imaging system (100) in a pre-data-acquisition-mode in which data acquisition by the antenna elements (114) is switched off,
a processor (130) for controlling the magnetic resonance imaging system (100), wherein an execution of the first set of machine executable instructions (150) causes the processor (130) to control the magnetic resonance imaging system (100) to generate in the pre-data-acquisition-mode acoustic sound using the magnetic field gradient system, wherein generating the acoustic sound comprises generating the alternating gradient magnetic field by the magnetic field gradient system.

2. The magnetic resonance imaging system (100) of claim 1, wherein the generating of the acoustic sound further comprises generating simultaneously with the alternating gradient magnetic field the constant main magnetic field using the main magnet (104).

3. The magnetic resonance imaging system (100) of any of the previous claims, wherein the memory (136) further comprises pulse sequence commands (148) configured for controlling the magnetic resonance imaging system (100) to acquire the magnetic resonance data (142) from the imaging zone (108), wherein the alternating of the gradient magnetic field in the pre-data-acquisition-mode is executed according to a gradient magnetic field alternating scheme defined by the pulse sequence commands (148).

4. The magnetic resonance imaging system (100) of claim 3, wherein amplitudes of pulses generated by the magnetic field gradient system in the pre-data-acquisition-mode are reduced relatively to amplitudes defined for the pulses by the gradient magnetic field scheme.

5. The magnetic resonance imaging system (100) of any of claims 3 to 4, wherein the amplitudes of pulses generated by the magnetic field gradient system are gradually raised until an amplitude threshold is reached.

6. The magnetic resonance imaging system (100) of claim 5, wherein the amplitude threshold is defined by a pulse with maximum amplitude defined by the gradient magnetic field scheme for the magnetic field gradient system.

7. The magnetic resonance imaging system (100) of any claims 3 to 6, wherein the acoustic sound generated using the gradient coils (110) further comprises a melodic tune.

8. The magnetic resonance imaging system (100) of any of the previous claims, wherein the volume of the acoustic sound is gradually raised until a volume threshold is reached.

9. The magnetic resonance imaging system (100) of claim 8, wherein the volume threshold is defined by the volume of the acoustic sound generated by the pulse with maximum amplitude defined by the gradient magnetic field scheme for the magnetic field gradient system.

10. The magnetic resonance imaging system (100) of any of the previous claims, wherein the magnetic resonance imaging system (100) further comprises a user interface (132) configured for manually tuning the acoustic sound generated by the magnetic field gradient system in the pre-data-acquisition-mode.

11. The magnetic resonance imaging system (100) of any of the previous claims, wherein the machine executable instructions stored in the memory (136) comprise a second set of machine executable instructions (152) for operating the magnetic resonance imaging system (100) in a data-acquisition-mode in which data acquisition by the antenna elements (114) is switched on, wherein an execution of the second set of machine executable instructions (152) causes the processor (130) to control the magnetic resonance imaging system (100) using the pulse sequence commands (148) to:
- acquire magnetic resonance data (142) from the imaging zone (108) using the antenna elements (114),
- reconstruct a magnetic resonance image (144) using the acquired magnetic resonance data (142).

12. A computer program product comprising machine executable instructions for execution by a processor (130) controlling a magnetic resonance imaging system (100), wherein the magnetic resonance imaging system (100) further comprises
a main magnet (104) configured for generating a spatially and temporally constant main magnetic field within an imaging zone (108),
a magnetic field gradient system comprising a set of gradient coils (110) configured for generating a spatially and temporally alternating gradient magnetic field within the imaging zone (108),
a radio-frequency system comprising a set of antenna elements (114) configured for acquiring magnetic resonance data (142) from the imaging zone (108),
wherein the machine executable instructions comprise a first set of machine executable instructions (150) for operating the magnetic resonance imaging system (100) in a pre-data-acquisition-mode in which data acquisition by the antenna elements (114) is switched off,
wherein an execution of the first set of machine executable instructions (150) causes the processor (130) to control the magnetic resonance imaging system (100) to generate in the pre-data-acquisition-mode acoustic sound using magnetic field gradient system, wherein generating the acoustic sound comprises generating the alternating gradient magnetic field by the magnetic field gradient system.

13. A method of operating a magnetic resonance imaging system (100), the magnetic resonance imaging system (100) comprising:
a main magnet (104) configured for generating a spatially and temporally constant main magnetic field within an imaging zone (108),
a magnetic field gradient system comprising a set of gradient coils (110) configured for generating a spatially and temporally alternating gradient magnetic field within the imaging zone (108),
a radio-frequency system comprising a set of antenna elements (114) configured for acquiring magnetic resonance data (142) from the imaging zone (108),
a memory (136) storing machine executable instructions comprising a first set of machine executable instructions (150) for operating the magnetic resonance imaging system (100) in a pre-data-acquisition-mode in which data acquisition by the antenna elements (114) is switched off,
a processor (130) for controlling the magnetic resonance imaging system (100),
wherein the method comprises executing the first set of machine executable instructions (150) by the processor (130) and generating in the pre-data-acquisition-mode acoustic sound using the magnetic field gradient system, wherein generating the acoustic sound comprises generating the alternating gradient magnetic field by the magnetic field gradient system.
